(19) Europäisches Patentamt
European Patent Office
Office européen des brevets

(11) EP 2 696 842 B1

(12) **EUROPEAN PATENT SPECIFICATION**

(45) Date of publication and mention of the grant of the patent:
**12.09.2018 Bulletin 2018/37**

(21) Application number: **11863489.8**

(22) Date of filing: **11.04.2011**

(51) Int Cl.:
*A61K 8/29* (2006.01)
*A61K 8/25* (2006.01)
*A61K 8/27* (2006.01)
*A61K 8/28* (2006.01)
*A61K 8/34* (2006.01)
*A61K 8/37* (2006.01)
*A61K 8/42* (2006.01)
*A61K 8/891* (2006.01)
*A61Q 1/02* (2006.01)
*A61Q 5/00* (2006.01)
*A61Q 19/00* (2006.01)
*A61K 8/31* (2006.01)
*A61Q 1/00* (2006.01)
*A61K 8/58* (2006.01)
*A61Q 5/02* (2006.01)
*A61Q 5/12* (2006.01)
*A61Q 9/02* (2006.01)
*A61Q 19/10* (2006.01)
*A61K 8/02* (2006.01)

(86) International application number:
**PCT/CN2011/000623**

(87) International publication number:
**WO 2012/139244 (18.10.2012 Gazette 2012/42)**

(54) **COSMETIC COMPOSITION**

KOSMETISCHE ZUSAMMENSETZUNG

COMPOSITION COSMÉTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**19.02.2014 Bulletin 2014/08**

(73) Proprietor: **The Procter & Gamble Company**
**Cincinnati, OH 45202 (US)**

(72) Inventors:
- **LI, Wei**
  **Beijing 102218 (CN)**
- **XIE, Yi**
  **Beijing 100102 (CN)**
- **HA, Robert (Bao Kim)**
  **Ohio 45011 (US)**

(74) Representative: **Engisch, Gautier**
**NV Procter & Gamble Services Company SA**
**IP Patent Department**
**Temselaan 100**
**1853 Strombeek-Bever (BE)**

(56) References cited:
**EP-A1- 2 169 040**
**WO-A1-02/056486**
**WO-A1-2005/044219**
**WO-A1-2009/071529**
**WO-A2-01/51017**
**WO-A2-99/66883**
**CN-A- 101 267 798**

- **NISHIKATA K: "A Natural Looking Make-up", COSMETICS & TOILETRIES, WHEATON, IL, US, vol. 112, 1 May 1997 (1997-05-01), pages 39-42,45, XP002120865, ISSN: 0361-4387**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### FIELD OF THE INVENTION

The present invention relates to a cosmetic composition providing color transformation upon its application onto skin, which comprises both a colored interference pigment and a non-interference pigment concentrate.

### BACKGROUND OF THE INVENTION

Cosmetic compositions are used to provide various benefits and meet different consumer needs. Those needs can include, for example, functional benefits such as skin moisturizing, wrinkle reduction, skin whitening and the like. Consumer needs can also include desirable consumer use experiences, for example, pleasant smell, smooth texture, appealing color and appearance of the composition, and can include more profound consumer experiences, for example, an interesting interaction regarding the use of a cosmetic composition.

Non-interference pigments have been widely used in cosmetic compositions. For example, titanium dioxide is used to achieve an acute whitening benefit. Such whitening skin coverage is highly desirable among Asian consumers. In addition, non-interference pigments are used to provide skin care products desired colors. For example iron oxide can provide a pink or red color. The non-interference pigments, whether they are hydrophilic or hydrophobic, when used in a cosmetic composition, are dispersed in a hydrophilic phase or a hydrophobic phase to achieve a homogenous dispersion and suspension in the cosmetic composition. For example, in an oil-in-water emulsion comprising titanium dioxide where the water phase is the continuous phase, it is normal in the art that hydrophilic titanium dioxide is used and dispersed in the water phase, not in the discontinuous oil phase.

It is known that a white oil-in-water emulsion type of skin moisturizer can be made pink in color, by incorporating a colored non-interference pigment iron oxide. The pink color is usually accepted as indicia by consumers that the product helps building a rosy and healthy skin complexion. Though consumers generally consider the color effects appealing, a cosmetic composition providing a more profound and sophisticated interaction with the consumer than the known single unchanged color effect is desirable.

In addition to non-interference pigments, interference pigments have also been widely used in cosmetic compositions, including skin care products, color cosmetic products and personal cleansing products, to provide glistening color effects, including colors such as white, gold, bronze, pearlescent and the like. Among the interference pigments, there are colored interference pigments, which provide a specific color instead of a flat wavelength response of pearlescent or white color. When the colored interference pigment particles achieve a parallel orientation on a surface, a maximized reflection color is perceivable to human eyes. This reflection color is different from the original color of the cosmetic composition, thus rendering a color transformation.

It is desirable to have a cosmetic composition that can provide a first color, and upon its application deliver an attractive color transformation, i.e., a color change from the first color to a second color, and meanwhile provide even coverage on the skin. This invention accomplishes these benefits.

While many others have sought to improve the color effect of the cosmetic composition, the benefit of this present invention was not met and not disclosed in the art. See, for example, WO patent application WO/2009/112970 and EP 2169040 A1.

### SUMMARY OF THE INVENTION

According to a first aspect, the present invention relates to a cosmetic composition comprising:

a) from about 0.1 % to about 5% of a colored interference pigment;
b) from about 0.1 % to about 10% of a non-interference pigment concentrate, wherein said concentrate is in the form of oil droplets comprising the non-interference pigment and oil;
c) from about 10% to about 90% of a cosmetically acceptable vehicle; wherein said cosmetic composition has a contrast ratio of 10 or less, and has a viscosity of at least about 40,000 mPa.s.

According to a second aspect, the present invention relates to a method of presenting a color transformation of the cosmetic composition during its application, comprising applying the present cosmetic composition.

### DETAILED DESCRIPTION OF THE INVENTION

While the specification concludes with claims particularly pointing out and distinctly claiming the invention, it is believed that the present invention will be better understood from the following description.

All percentages, parts and ratios are based upon the total weight of the compositions of the present invention, unless otherwise specified. All such weights as they pertain to listed ingredients are based on the active level and, therefore, do not include carriers or by-products that may be included in commercially available materials.

As used herein, "comprising" means that other steps and other ingredients which do not affect the end result can be added. This term encompasses the terms "consisting of" and "consisting essentially of".

As used herein, the term "cosmetic composition" refers to what is suitable for topical application on mammalian keratinous tissue. Products contemplated by the term "cosmetic product" include, but are not limited to, skin care composition, hair conditioner, shaving cream, personal cleansing product and color cosmetic composition.

As used herein, the term "interference pigment" means a pigment that exhibits iridescence, an optical phenomenon in which hue changes according to the angle from which the surface is viewed.

As used herein, the term "D0.99" or "D99" is a measure of particle size relating to particle size distribution, and means that 99% of the particles have a particle size in a certain range.

As used herein, the term "cosmetically acceptable vehicle" is an oil-in-water emulsion.

As used herein, the term "non-interference pigment concentrate" means the non-interference pigment exists in the present cosmetic composition in a concentrated form, such as in the form of oil droplets comprising the non-interference pigment and oil.

As used herein, the term "oil droplets" means the blend of oil and non-interference pigment, which exists stably as a discontinuous phase in the cosmetically acceptable vehicle. It is made by carefully dispersing the blend of oil with the non-interference pigment into the cosmetically acceptable vehicle.

As used herein, the term "first oil" and "second oil" are both oils selected from the same group of hydrocarbon oil, silicone oil, silicone wax, or combinations thereof. "First oil" stands for the oil used in forming oil droplets, while "second oil" stands for the oil used in the carrier. First oil and second oil can include a mixture of various oils. First oil and second oil can be partially overlapping, identical, or totally different from the other.

As used herein, the term "first oil droplet" is the oil droplet comprising the first oil and non-interference in present composition, which keeps the contrast ratio of the present composition to be 10 or less.

As used herein, the term "contrast ratio" is an optical transparency measurement. The higher contrast ratio value a cosmetic composition has, the less transparent the composition is.

As used herein, the term "color transformation" means a color change of the cosmetic composition, which happens when the cosmetic composition is applied onto the skin, as compared to the cosmetic composition's original bulk color as perceived when the composition is stored in a vessel. In one embodiment, the color transformation is transient and shows only when the cosmetic composition is applied onto skin in one direction and spread into a thin layer. The color transformation may vanish after the cosmetic composition being applied on the skin in multiple back and forth stokes. There are a number of ways to measure the color transformation, including skilled panel's perception, and Hunter color measurement. Panel perception test is used for an embodiment of the present invention.

As used herein, the term "room temperature" means about 25°C.

The present cosmetic composition comprises a colored interference pigment, a non-interference pigment concentrate and a cosmetically acceptable vehicle, where the cosmetic composition has a contrast ratio of 10 or less, and has a viscosity of at least about 40,000 mPa.s. It has been surprisingly found that the present cosmetic composition exhibits a perceivable and desirable color transformation, when it is applied onto skin, in the situation when the colored interference pigments exist in the same cosmetic composition with the non-interference pigment concentrate. Moreover, non-interference pigment within the non-interference concentrate can be easily released during application to achieve its own effect, for example, acute whitening.

It is now possible to deliver the colored interference pigments and the non-interference pigments in the same cosmetic composition, and achieve a color transformation, which has not been known in the art before the present invention. It is believed that the fine particle size and thereby the large total surface area of dispersed non-interference pigments cause the non-interference pigments to scatter the light essential for a color transformation away from the colored interference pigment, thus preventing the color transformation. The present invention solves the problem by concentrating the non-interference pigments into oil-droplets. Without wishing to be bound by theory, it is believed that by concentrating the non-interference pigment into oil droplets, the non-interference pigment is prevented from scattering the light necessary for the colored interference pigment to confer a color change. The degree of concentration sufficient for the cosmetic composition comprising both the colored interference pigment and the non-interference pigment concentrate to show color transformation is measured by the contrast ratio of the cosmetic composition. It has been surprisingly found that by carefully incorporating non-interference oil droplets into a cosmetic composition, and controlling the contrast ratio to 10 or less, the present cosmetic composition shows the desired color transformation from the interference pigment and skin coverage benefit from the non-interference pigment.

The cosmetic composition of the present invention, during its application, provides a noticeable color change, thus more efficiently establish an interaction with the consumers that the functional benefit, e.g. a color change from white to pink, is a strong indicia during application for benefiting the consumer's skin toward a healthy rosy complexion,

i.e. "pink from within". This color transformation, in the minds of the consumers, is more powerful than a single unchanged color of the product itself, in that it can provide enhanced, vivid, and instant demonstration, and thus increased believability among consumers of the actual functional benefit of the cosmetic composition, and improved purchasing intent among consumers. The present cosmetic composition can provide such a color transformation when it is applied via a stroke in one direction on a skin surface.

COLORED INTERFERENCE PIGMENT

The colored interference pigment in the present invention is a pigment prepared by coating the surface of a particle substrate material with a thin film. The particle substrate material is generally platelet in shape. The colored interference pigment of the present invention is a crystalline or glassy solid, transparent or translucent compound capable of reflecting and refracting light to produce the desired iridescent effect. In a preferred embodiment of the present invention, the colored interference pigment is uniformly dispersed throughout the cosmetic acceptable carrier of the present cosmetic composition. The present cosmetic composition comprises from about 0.1% and 1% to about 2.5% and 5% of an interference pigment.

In one embodiment, colored interference pigments show an absorbance minimum of from about 380 to about 750 nm in the visible range of the light spectrum. In contrast, pearlescent pigments have a flat wavelength response in the visible light spectrum. As a result of such contrast, colored interference pigments reemit light at specific wavelengths in the visible spectrum, whilst pearlescent pigments reemit "white" light without specific color. Thus, according to the present invention, the colored interference pigment shows one or more absorbance minima of from about 380 to about 750 nm in the visible range of the light spectrum.

The colored interference pigments are selected from, but not limited to, a group of interference pigments consisting of: colored interference pigments showing an absorption minimum of about 570-590 nm (violet color); colored interference pigments showing at least an absorption minimum of about 590-620 nm (blue color); colored interference pigments showing at least an absorption minimum of about 620-750 nm (green color); colored interference pigments showing at least an absorption minimum of about 380-450 nm (yellow color); colored interference pigments showing at least an absorption minimum of about 450-495 nm (orange color); colored interference pigments showing at least an absorption minimum of about 495-570 nm (red color) or mixture thereof.

A wide variety of particle substrates are useful herein. Non-limiting examples are natural mica, synthetic mica, graphite, talc, kaolin, alumina flake, bismuth oxychloride, silica flake, glass flake, ceramics, titanium dioxide, $CaSO_4$, $CaCO_3$, $BaSO_4$, borosilicate and combinations thereof, preferably mica, silica and alumina flakes.

A wide variety of thin films are useful herein. Non-limiting examples are $TiO_2$, $Fe_2O_3$, $SnO_2$, $Cr_2O_3$, ZnO, ZnS, ZnO, SnO, $ZrO_2$, $CaF_2$, $Al_2O_3$, BiOCl, and combinations thereof or in the form of separate layers, preferably $TiO_2$, $Fe_2O_3$, $Cr_2O_3$, and $SnO_2$. The thin film layers can contain a dye. In one embodiment, the interference pigment is a $TiO_2$ coated mica, specifically, under the name of Timiron Splendid Violet from MERCK.

The interference color is a function of the thickness of thin film. In one embodiment, the thickness of the layer coating the substrate is at least about 60 nm in the colored interference pigments. Colored interference pigments are different from commonly known pearlescent pigments which exhibit only a white visual effect as this white visual effect is due to the thickness of the $TiO_2$ layer of the pearlescent pigments being less than 60 nm.

The thickness required for a specific color may be different depending on materials. For example, in the case of $TiO_2$, a film layer comprises a thickness of about 60nm to about 80nm for a yellow color, about 80nm to about 100nm for a red color, about 100nm to about 130nm for a blue color, and about 130nm to about 160nm for a green color. In addition to the interference color, other transparent absorption pigments can be precipitated on top of or simultaneously with the thin film layer. Common materials are iron oxide, ferric ferrocyanide, chromium oxide or carmine. In general, preferred interference colors include but are not limited to gold, red, green, violet, blue and combinations thereof.

Without wishing to be bound by theory, it is believed that the characteristic of single reflection color of each pigment is an optical effect caused by light interference. Therefore, through controlled thickness of the metal oxide layer, all colors of the rainbow can be achieved.

Moreover, by using different particle sizes, with mica as a base, colored interference pigments can exhibit different luster effects. Particle size is measured across the largest diameter. Platelet particles are such that two dimensions of the particle, e.g. length and width are at least 5 times the third dimension, e.g. depth or thickness. Other crystal shapes like cubes or needles or other crystal shapes do not display pearlescent effect. Many colored interference pigments like mica are natural minerals having monoclinic crystals.

Volume particle size and particle size distribution are measured using the Hydro 2000G equipment available from Malvern Instruments Ltd. Particle size has a role in stabilization of the pigments. The smaller the particle size and distribution are, the more easily the particles are suspended. However, as the particle size of the colored interference pigments is decreased, so the efficacy of the pigments is decreased.

The colored interference pigments have D0.99 volume particle size of from about 0.1 $\mu$m, 0.5$\mu$m, and 1$\mu$m to

about 20μm, 25μm, and 50 μm. In a preferred embodiment, the colored interference pigments have D0.99 volume particle size of less than about 50 μm.

Commercially available colored interference pigments suitable are available from BASF under the trade-names Lumina Gold, Lumina Turquoise, Lumina Green, Lumina Red, Lumina Red Blue, Lumina Aqua Blue, Rutile Fine Lilas, Mearlin Dynacolor Green Blue, Mearlin Dynacolor Blue Green, Mearlin Dynacolor Green, Exterior Red, Exterior Blue, Exterior Gold.

Other commercially available colored interference pigments are available from Merck under the trade-names of Timiron Super Blue, Timiron Gold Plus, Iriodin Rutile Fine Red, Iriodin Rutile Fine Lilac, Iriodin Rutile Fine Green, Iriodin Rutile Fine Blue, Iriodin Rutile Fine Gold, and Iriodin Rutile Red Pearl. Non-limiting examples of the colored interference pigments useful herein also include those supplied by Persperse, Inc. under the trade name Prestige, Flonac; supplied by MERCK, under the trade name of Timiron, for example Timiron Splendid Violet; supplied by EMD Chemicals, Inc. under the trade name of Colorona, Dichrona and Xirona; and supplied by Engelhard Co. under the trade name of Flamenco, Timica, Duochrome; and supplied by Kobo Products Inc, under the trade name of KTZ, for example KTZ Xian Vista-11S2, KTZ Shimmer Red.

NON- INTERFERENCE PIGMENT CONCENTRATE

The present cosmetic composition further comprises a non-interference pigment concentrate. Through concentrating non-interference pigments, the present cosmetic composition is able to provide not only the acute benefit of the non-interference pigment, but also the attractive color change brought by interference pigment. The concentrate is in a form of "first oil droplets" comprising "first oil" and a non-interference pigment.

NON-INTERFERENCE PIGMENT

The non-interference pigment can be water-dispersible and/or oil-dispersible. Water or oil dispersibility may be inherent to the particle or may be imparted by coating the particles with materials to impart a hydrophilic or hydrophobic surface property to the particles. For example, hydrophilic coatings may comprise an amino acid, aluminum oxide or aluminum silicate. Exemplary hydrophobic coatings may comprise organosilicone compounds or metal soaps such as aluminum stearate, aluminum laurate, and zinc stearate. The present cosmetic composition comprises from about 0.1 %, 0.5% and 1% to about 2%, 5% and 10% of a non-interference pigment concentrate which comprises about 0.09%, 0.3% to about 1% and 3% of non-interference pigments.

Preferred particulate materials are those having a primary particle size of from about 100nm, 200nm, 250nm to about 300 nm, 400nm and 1000nm. Primary particle size can be determined using the ASTM Designation E20 - 85 "Standard Practice for Particle Size Analysis of Particulate Substances in the Range of 0.2 to 75 Micrometers by Optical Microscopy", ASTM Volume 14.02, 1993, incorporated herein by reference.

In one embodiment, the non-interference pigment useful herein can be those pigments providing a whitening benefit. Typical whitening pigments include pigment-grades of titanium dioxide, zinc oxide, talc, mica and combinations thereof.

Non-limiting examples of suitable whitening non-interference pigments are available from Warner Jenkinson, coded C-9729, which is a hydrophobic, dimethicone treated, anatase form $TiO_2$; from U. S. Cosmetics under the name Tronox $TiO_2$ series, e.g., AT-T-CR837, which is a hydrophilic, rutile, amino acid treated $TiO_2$; AT-T-328, a hydrophilic, anatase, amino acid treated $TiO_2$; and SAT-T CR837, a rutile $TiO_2$; from Kobo under the name Tronox $TiO_2$ series, e.g., ST490, a rutile, silane treated $TiO_2$; and from Kobo under the name of FA65UMLO, FA65USI, which are hydrophobic titanium dioxide.

The particulate materials are available in an essentially neat, powdered form or predispersed in various types of dispersants, including, but not limited to, isopropyl isostearate, isopropyl palmitate, methyl isostearate, Finsolv TN, cylcomethicone, and cyclomethicone and dimethicone copolyols. Non-limiting examples are available from Kobo under the name of GLY45GYAP, GLW75PFAP-MP, which are aqueous dispersions of $TiO_2$.

Examples of commercially available zinc oxide pigment include Z-Cote HP1 from BASF CORP. Examples of commercially available talc pigment include ZnO-USP1-MS3 from Kobo Products Inc.

In one embodiment, the non-interference pigment also includes a colored pigment in addition to a whitening pigment, including but not limited to, hydrophobic iron oxide pigments selected from yellow hydrophobic iron oxide pigments, red hydrophobic iron oxide pigments, black hydrophobic iron oxide pigments or mixtures thereof. Yellow iron oxide pigment is also known as goethite, ferric oxide hydrate or CI 77492. Red iron oxide pigment is also known as haematite, ferric oxide and CI 77491. Black iron oxide pigment is also known as magnetite, ferrous-ferric oxide and CI 77499. Examples of commercially available hydrophobic iron oxide pigments include FA50EYSI, FA55ERSI and FA60EBSI from Kobo products.

FIRST OIL DROPLETS

The non-interference pigment concentrate is in the form of first oil droplets comprising a first oil and a non-interference pigment. Useful first oil herein can be a single oil ingredient or mixtures of several oil ingredients. Useful first oil in the present invention may exhibit one or more of the following characteristics - it may be saturated or unsaturated, have a straight or branched chain or a cyclic structure. The "first oil" droplets of the present cosmetic composition can comprise from about 0.01%, 0.1% and 1% to about 2%, 5% and 7% of a "first oil" and from about 0.09%, 0.2% and 0.5% to about 1%, 1.5% and 3% of the non-interference pigment, by weight of the cosmetic composition.

The first oil is selected from the group of oils consisting of hydrocarbon oil, silicone oil, silicone wax, or combinations thereof. Suitable hydrocarbon oils may be selected from a group consisting of fatty acid, fatty acid esters, branched esters of diglycerin or triglycerin, the esters or 1,2,3,4 butane triol or erythritol, dierythritol or trierthyritol, or combinations thereof. In one embodiment, hydrocarbon oils comprise behenyl alcohol, stearyl alcohol, cetearyl alcohol, vitamine E acetate, olive oil, isopropyl isostearate, isopropyl palmitate, erythrityl triethylhexanoate (available as Salacos E-38 from Nisshin Oilio), polyglyceryl-2 triisostearate (available as Cosmol 43V from Nisshin Oilio), diethyl hexyl carbonate (available as Tegosoft DEC from Degussa), dicapryl ether (available as Cetiol OE from Cognis AG), dicapryl carbonate (available as Cetiol CC from Cognis AG), isononyl isononanoate (available as Lanol 99 from Seppic), tridecyl neopentanoate (supplied as Ceraphyl 55 from International Speciality Products), or mixtures thereof. Examples of hydrocarbons oils include polydecanes such as isododecane and isodecane (e.g., Permethyl-99A which is available from Presperse Inc.) and the C7-C15 isoparaffins (such as the Isopar Series available from Exxon Chemicals).

Suitable silicone oils include linear polymethylsiloxanes and, preferably, high molecular weight dimethicones. Examples of commercially available linear polymethylsiloxanes include DC 200, DC 200 Fluid 20Cst, DC 200 Fluid 100Cst, and DC 200 Fluid 350Cst from Dow Corning Corporation. Preferably, the linear silicone oil is a linear polymethylsiloxane.

The silicone oil may be selected from cyclic silicone oils corresponding to the formula:

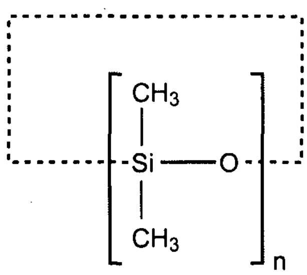

wherein n is from 3 to 7,
linear silicone oils corresponding to the formula

$$(CH_3)_3Si\text{-}O\text{-}[Si(CH_3)_2\text{-}O]m\text{-}Si(CH_3)_3$$

wherein m is from 1 to 20, preferably from 3 to 12.

In one embodiment, the cyclic silicone oil is selected from cyclopentasiloxane, cyclohexasiloxane or mixture thereof. Examples of commercially available cyclic silicone oils include DC 244, DC 245, DC 344, and DC 345 from Dow Corning Corp.; SF-1204 and SF-1202 Silicone Fluids from Momentive Performance Materials; GE 7207 and 7158 from General Electric Co.); and, SWS-03314 from SWS Silicones Corp. Other suitable silicone oil used herein can also include for example DC1503, a mixture of dimethicone and dimethiconol.

Suitable silicone wax includes C24-45 alkyl methicones, for example under the name of DC2503 from Dow Corning Corp.

The present first oil droplets together with the colored interference pigment exist in the cosmetic acceptable carrier of the present cosmetic composition.

The first oil droplets can be prepared as following. First, a homogenous blend of the non-interference pigment with one or more first oil is made at a temperature higher than the melting point of the first oils, and the same time a cosmetic acceptable carrier is prepared according to conventional preparation method. Then the blend is cooled down and incorporated into the carrier through a gentle mixing to form said oil droplets. By "gentle mixing", it means that the shear force applied during mixing will not be too strong to break the oil droplet into a very fine droplet size so that the contrast ratio of present cosmetic composition comprising the oil droplets is greater than 10. The shear force can be determined by a number of factors including the type of a blender used for mixing, the viscosity of the blended composition, and the speed of mixing. In one embodiment, when a RW11B blender from RW11B is used for mixing, under various

viscosity value of the present cosmetic composition, it is preferred that the mixing speed is no higher than 500rpm, no higher than 300rpm, no higher than 200rpm, no higher than 100 rpm, or no higher than 50rpm.

COSMETICALLY ACCEPTABLE VEHICLE

The present cosmetic composition comprises from about 10% to about 90% of a cosmetically acceptable vehicle. The appropriate vehicle is selected by one skilled in the art depending on the application of the cosmetic composition. The cosmetically acceptable vehicle and the resultant cosmetic compositions herein is formulated in the form of an oil-in-water emulsion. The non-interference pigment concentrate of the present invention is in the form of first oil droplets, the cosmetically acceptable vehicle is a second oil-in-water emulsion. The second oil in the second oil-in-water emulsion is selected from the same pool of oil ingredients as described in previous paragraphs under FIRST OIL DROPLETS section. That means the second oil is also selected from the same group of oils consisting of hydrocarbon oil, silicone oil, silicone wax, or combinations thereof. The second oil presents in the second oil-in-water emulsion is in the range of from about 1%, 2% or 5% to about 10%, 30% or 50 % based on the total weight of the cosmetic composition.

In one embodiment, in the preparation of the present cosmetic composition, the first oil has a melting point of no less than 27°C, preferably no less than 35°C; while the second oil in the second oil-in-water emulsion has a melting point of around 32°C-40°C or higher. These melting point arrangement for both first oil and second oil is preferred in that it allows the processing convenience, when the blend of first oils and non-interference pigment is mixed with the second oil-in-water emulsion in order to form the first oil droplets, and helps stabilize the discontinuous phase in the second oil-in-water emulsion by preventing coalesce between numerous first oil droplets and between first oil droplet and the discontinuous phase in the emulsion.

The second oil-in-water emulsion in the present cosmetic composition comprises an emulsifier which is suitable for use in the cosmetic industry. Generally, the emulsifier helps disperse and suspend the oil within the continuous aqueous phase of the second oil-in-water emulsion. In one embodiment, the emulsifier is selected from nonionic emulsifiers, cationic emulsifiers, anionic emulsifiers, zwitterionic emulsifiers, amphoteric emulsifiers, or combinations thereof. In another embodiment, the emulsifier is a nonionic emulsifier. Suitable emulsifiers may be found in McCutcheon's, Detergents and Emulsifiers, North American Edition (1986), published by Allured Publishing Corporation. Examples of commercially available emulsifiers include Myrj 59 (PEG-100 stearate) from Uniquema Americas; Arlacel 60 (Sorbitan Stearate) from Uniquema Americas, and Emulgade PL 68/50 (cetearyl glucoside and cetearyl alcohol) from Cognis Deuschland GmbH & CO. KG. In one embodiment, the present cosmetic composition comprises from about 0.1% to about 7%, preferably from about 0.1% to about 2% of an emulsifier. In another embodiment, the present cosmetic composition comprises emulsifiers selected from the group consisting of sorbitan stearate, sucrose cocoate, cetearyl glucoside and cetearyl alcohol, stearic acid, PEG-100 stearate, and combinations thereof.

The second oil-in-water emulsion of the present cosmetic composition optionally comprises a thickener. The composition of the present invention comprise from about 0.01% or 0.1% to about 7% or 10% of a thickener. The viscosity can be measured using the viscosity measurement method. The present cosmetic composition has a viscosity of at least 40,000 cps, and preferably, has a viscosity from about 40,000 cps to about 200,000 cps.

Non-limiting examples of thickeners useful herein include carboxylic acid polymers such as the carbomers (such as those commercially available under the tradename Carbopol 900 series from B.F. Goodrich; e.g., Carbopol 980). Other suitable carboxylic acid polymeric agents include copolymers of C10-30 alkyl acrylates with one or more monomers of acrylic acid, methacrylic acid, or one of their short chain (i.e., C1-4 alcohol) esters, wherein the crosslinking agent is allyl ether of sucrose or pentaerytritol. These copolymers are known as acrylates/C10-30 alkyl acrylate crosspolymers and are commercially available as Carbopol 1342, Carbopol 1382, Pemulen TR-1, and Pemulen TR-2, from B.F. Goodrich.

Other non-limiting examples of thickener include crosslinked polyacrylate polymers including both cationic and nonionic polymers.

Still other non-limiting examples of thickener include the polyacrylamide polymers, especially nonionic polyacrylamide polymers including substituted branched or unbranched polymers. More preferred among these polyacrylamide polymers is the nonionic polymer given the CTFA designation polyacrylamide and isoparaffin and laureth-7, available under the trade name Sepigel 305 from Seppic Corporation (Fairfield, NJ). Other polyacrylamide polymers useful herein include multi-block copolymers of acrylamides and substituted acrylamides with acrylic acids and substituted acrylic acids. Commercially available examples of these multi-block copolymers include HYPAN SR150H, SS500V, SS500W, SSSA100H, from Lipo Chemicals, Inc., (Patterson, NJ).

Another non-limiting class of thickener useful herein is the polysaccharides. Non-limiting examples of polysaccharide include those selected from cellulose, and cellulose derivatives. Preferred among the alkyl hydroxyalkyl cellulose ethers is the material cetyl hydroxyethylcellulose, which is the ether of cetyl alcohol and hydroxyethylcellulose, sold under the tradename of Natrosel CS Plus from Aqualon Corporation (Wilmington, DE). Other useful polysaccharides include scleroglucans which are a linear chain of (1-3) linked glucose units with a (1-6) linked glucose every three units,

a commercially available example of which is Cleargel CS 11 from Michel Mercier Products Inc. (Mountainside, NJ).

Another non-limiting class of thickener useful herein is gums. Non-limiting examples of gums useful herein include hectorite, hydrated silica, xantham gum, and combinations thereof.

Yet another non-limiting class of thickener useful herein is the modified starches. Acrylate modified starches such as Waterlock from Grain Processing Corporation may be used. Hydroxypropyl starch phosphate, trade name Structure XL from National Starch is another example of a useful modified starch, and other useful examples include Aristoflex HMB (Ammonium Acrylodimethyltaruate/Beheneth-25 Methacrylate Crosspolymer) from Clariant.

In one embodiment, the cosmetic composition comprises thickeners selected from the group consisting of acrylate polymer, carbomer, fatty alcohol, and combinations thereof.

The cosmetic compositions of the present invention may include at least one skin care active. In any embodiment of the present invention, the actives useful herein can be categorized by the benefit they provide or by their postulated mode of action. However, it is to be understood that the actives useful herein can in some instances provide more than one benefit or operate via more than one mode of action. Therefore, classifications herein are made for the sake of convenience and are not intended to limit the active to that particular application or applications listed. Non-limiting examples of these additional ingredients includes; particular materials to modify skin feel or appearance; anti-acne actives; oil-soluble beta-hydroxy acids such as salicylic acid and derivatives thereof; flavonoid compounds; anti-inflammatory agents; anti-cellulite agents; exfoliating actives; anti-oxidant/radical scavengers; tanning actives; skin soothing or skin healing actives such as panthenoic acid derivatives (including panthenol, dexpanthenol, ethyl panthenol), aloe vera, allantoin, bisabolol, niacinamide, sodium ascorbyl phosphate, glucosyl hesperidin and dipotassium glycyrrhizinate; antimicrobial or antifungal actives.

METHOD OF PREPARATION

Because the non-interference pigment concentrate is in the form of first oil droplets and the cosmetically acceptable vehicle is an second oil-in-water emulsion, it can be prepared by: (i) following a conventional preparation method to prepare the emulsion comprising the colored interference pigment, which involves mixing of the ingredients in one or more steps to a relatively uniform state, with or without heating, cooling, application of vacuum, and the like, (ii) preparing a blend of first oil with the non-interference pigment by mixing the two at a temperature of above the melting point of the first oil used, (iii) cooling down the blend and mix it with the second oil-in-water emulsion to disperse the blend into first oil droplets. Steps (i) may be carried out before or simultaneously to steps (ii) and (iii).

CONTRAST RATIO MEASUREMENT

Compositions of the present invention have a contrast ratio of 10 at most, more preferably 8 at most, more preferably 7 at most. Contrast ratio is used to measure the optical transparency of the composition, and can be determined by the following Contrast Ratio Method, using an standard opacity chart (Form 2A from Leneta Company of Manwah, NJ or the equivalent thereof), and a chromo meter (e.g., Color-EYE 3100, GretagMacbeth, C illuminant, 0 degree viewing angle) equipped with a specular component, commercially available from the Minolta Camera Co. of Ramsey, NJ and described in the chromameter manual, version 3.0; 1988, incorporated herein by reference.

The opacity chart is black at the top and white at the bottom in equal portions, with the colors being separated along a line parallel to the horizon. 4 grams of a test product are applied in about a 1/2" X 3" area at the top of the opacity chart. The product is drawn down the length of the card, through both halves and generally symmetrically, into a thin film using a 1 mil film applicator (e.g., as commercially available from BYK Gardner of Columbia, Maryland) or the equivalent thereof. The film is allowed to dry for 24 hours under conditions of 25±1°C, 1 atmosphere.

Using the chromameter, the Y tristimulus value (i.e., the XYZ color space of the film) of the product film is measured and recorded. The Y tristimulus value is measured in three different areas of the product film over the black section of the opacity chart, and also in three different areas of the product film over the white section of the opacity chart. Areas which are measured are those having an even distribution of product over the area.

The contrast ratio is calculated as the mathematical average of the three Y tristimulus values over the black areas, divided by the mathematical average of the three Y tristimulus values over the white areas, times 100:

$$\text{Contrast Ratio} = \frac{\text{Average (three } Y_{black})}{\text{Average (three } Y_{white})} \quad X \quad 100$$

COLOR TRANSFORMATION MEASUREMENT

The color transformation of the cosmetic composition can be measured by a panel test. Directed questions are raised to the panelist for judging if the color transformation of the cosmetic product is perceived. The test products are provided to the panelists randomly. The test can be performed indoors under daylight lamp following the instructions: 1) wash antebrachium with cleansing foam, and outline a product usage area. (antebrachium, 3 $cm^2$); 2) apply pre-weighted, for example 0.2g, test product on said area, spreading the product in one direction, in one stroke; 3) after application, panelists are requested to immediately observe said application area, and tell whether there is color transformation perceivable with naked eye on the area; 4) wipe off the product, clean antebrachium using soap.

VISCOSITY MEASUREMENT

Viscosity of the present cosmetic composition can be measured using any commercially available viscometer. For example, the Brookfield DV-II+ PRO Digital Viscometer with the shear speed of 5 rpm at 25°C, 93# needle is used herein for the purpose of this measurement, and the viscosity number is obtained by averaging 4-5 times of repeated measurements.

METHOD OF USE

The present cosmetic composition can be used suitably for topical application on mammalian keratinous tissue, including, but are not limited to skin care composition, hair conditioner, shaving cream, personal cleansing product, color cosmetic composition.

EXAMPLES

The following examples further describe and demonstrate embodiments within the scope of the present invention. The examples are given solely for the purpose of illustration and are not to be construed as limitations of the present invention, as many variations thereof are possible without departing from the spirit and scope of the invention. Examples 1-8 represent non-limiting examples of cosmetic care compositions according to the present invention, while comparative Examples 1-4 are those not according to the present invention.

Table 1: Examples 1-8

| No | Component | Ex. 1 | Ex. 2 | Ex. 3 | Ex. 4 | Ex.5 | Ex.6 | Ex. 7 | Ex. 8 |
|---|---|---|---|---|---|---|---|---|---|
| Oil-in-water emulsion | | | | | | | | | |
| 1 | DI Water | Qsp | Qsp | Qsp | Qsp | Qsp | Qsp | Qsp | Qsp |
| 2 | Glycerin | 10 | 10 | 10 | 10 | 10 | 10 | 10 | 10 |
| 3 | Carbomer *1 | 0.65 | 0.65 | 0.65 | 0.65 | 0.65 | - | - | 0.65 |
| 4 | polyacrylamide & C13-14 Isoparaffin and Laureth-7*2 | - | - | - | - | - | 3 | - | - |
| 5 | Cetearyl Glucoside and Cetearyl Alcohol *3 | 1 | 1 | 1 | 1 | 1 | 1 | 1 | 1 |
| 6 | Isopropyl Isostearate | 6 | 6 | 6 | 6 | 6 | 6 | 6 | 6 |
| 7 | Behenyl Alcohol | 1 | 1 | 1 | 1 | 1 | 1 | 1 | 1 |
| 8 | Stearyl Alcohol | 1 | 1 | 1 | 1 | 1 | 1 | 1 | 1 |
| 9 | Stearic Acid | 0.5 | 0.5 | 0.5 | 0.5 | 0.5 | 0.5 | 10 | 0.5 |
| 10 | Ethylparaben | 0.2 | 0.2 | 0.2 | 0.2 | 0.2 | 0.2 | 0.2 | 0.2 |
| 11 | Propylparaben | 0.1 | 0.1 | 0.1 | 0.1 | 0.1 | 0.1 | 0.1 | 0.1 |
| 12 | Vitamin E Acetate | 1 | 1 | 1 | 1 | 1 | 1 | 1 | 1 |
| 13 | Sodium Hydroxide | 0.25 | 0.25 | 0.25 | 0.25 | 0.25 | - | 0.35 | 0.25 |
| 14 | Hydrophilic TiO2*4 | 0 | 0.005 | - | 0 | 0 | 0 | 0 | 0.08 |
| 15 | Hydrophobic iron oxide *5 | - | - | 0.005 | - | - | - | - | - |

(continued)

| No | Component | Ex. 1 | Ex. 2 | Ex. 3 | Ex. 4 | Ex.5 | Ex.6 | Ex. 7 | Ex. 8 |
|---|---|---|---|---|---|---|---|---|---|
| Oil-in-water emulsion | | | | | | | | | |
| 16 | Hydrophilic Interference Mica *6 | 0.5 | 2 | 2 | - | 2.5 | 2.5 | 3 | 2 |
| 17 | Hydrophobic Interference Mica *8 | - | - | - | 2 | - | - | - | - |
| 18 | Benzyl Alcohol | 0.2 | 0.2 | 0.2 | 0.2 | 0.2 | 0.2 | 0.2 | 0.2 |
| 19 | Perfume | 0.15 | 0.15 | 0.15 | 0.15 | 0.15 | 0.15 | 0.15 | 0.15 |
| 20 | Niacinamide | 3 | 3 | 3 | 3 | 3 | 3 | 3 | 3 |
| 21 | Sodium Ascorbyl Phosphate | 1 | 1 | 1 | 1 | 1 | 1 | 1 | 1 |
| 22 | Glucosyl Hesperidin | 0.1 | 0.1 | 0.1 | 0.1 | 0.1 | 0.1 | 0.1 | 0.1 |
| Oil droplets | | | | | | | | | |
| 23 | Cyclopentasiloxane *9 | 0.03 | - | - | 0.43 | - | - | - | |
| 24 | Stearyl dimethicone*10 | 0.06 | 0.06 | - | - | - | 0.06 | - | 0.06 |
| 25 | Dimethicone (and) Dimethiconol *11 | - | 0.19 | - | - | - | 0.19 | 0.19 - | 0.19 |
| 26 | Isopropyl Isostearate | - | - | - | - | 0.25 | - | 0.25 | - |
| 27 | Olive Oil *12 | - | - | 0.2 | - | - | - | - | - |
| 28 | hydrophobic $TiO_2$ *13 | 0.2 | 1 | - | 1.5 | 1 | 1 | 1 | 1 |
| 29 | hydrophobic ZnO *14 | - | - | 1 | - | - | - | - | |

*1 Carbopol 980 from Noveon
*2 Sepigel 305 from Seppic
*3 Cetearyl Glucoside and Cetearyl Alcohol: Emulgade from Cognis
*4 GLW45GYAP from Kobo Products Inc
*5 FA50EYSI from Kobo Products Inc
*6 Timiron® splendid violet from Merck
*8 KTZ Xian Vista - 11S2 from Kobo Products Inc
*9 Cyclopentasiloxane: D5 from Dow Corning Corporation
*10 DC2503 from Dow Corning Corporation
*11 DC1503 from Dow Corning Corporation
*12 AEC Olive Oil from A & E Connock (Perfumery & Cosmetics) Ltd.
*13 SA Titanium Dioxide CR-50 from MIYOSHI KASEI CO LTD
*14 Z-Cote HP1 from BASF CORP

The compositions of Examples 1-8 are suitably made as following:

1) Prepare a mixture of water soluble components 1, 3-5, 10-11, 13-14 and 18;
2) Prepare a mixture of oil soluble components comprising 6-9, 12, 15, and 19;
3) Mix those mixtures from step 1) and 2) at a temperature of around 75°C to effect emulsification, and cool it down to around 50°C after a homogenous mixture is achieved, then add a mixture of those less heat-resistant components including water soluble components 2, 16 and 20-22, and components 17, and keep mixing to get an homogenous second oil-in-water emulsion;
4) Prepare a blend of separate first oils including components 23-27 with hydrophobic non-interference pigment including components 28-29. If one or more of the first oils are in solid before blending, heating of the first oils to higher than melting point and keeping the first oils as a liquid can be used;
5) Pour the blend of step 4) into the second oil-in-water emulsion of step 3) at 30°C, and stirred for 10 minutes at a speed of 50-100 rpm/min using a blender coded RW11B from IKA. The first oil droplets are then formed within the second oil-in-water emulsion.

Comparative Examples 1-4

Table 2: Comparative Example 1-4

| No | Component | Com. Ex. 1 | Com Ex. 2 | Com Ex. 3 | Com EX 4 |
|---|---|---|---|---|---|
| Oil-in-water emulsion | | | | | |
| 1 | DI Water | Qsp | Qsp | Qsp | Qsp |
| 2 | Glycerin | 10 | 10 | 10 | 10 |
| 3 | Carbomer *1 | 0.65 | 0.65 | 0.65 | 0.65 |
| 4 | Cetearyl Glucoside and Cetearyl Alcohol | 1 | 1 | 1 | 1 |
| 5 | Isopropyl Isostearate | 6 | 6 | 6 | 6 |
| 6 | Behenyl Alcohol | 1 | 1 | 1 | 1 |
| 7 | Stearyl Alcohol | 1 | 1 | 1 | 1 |
| 8 | Stearic Acid | 0.5 | 0.5 | 0.5 | 0.5 |
| 9 | Ethylparaben | 0.2 | 0.2 | 0.2 | 0.2 |
| 10 | Propylparaben | 0.1 | 0.1 | 0.1 | 0.1 |
| 11 | Vitamin E Acetate | 1 | 1 | 1 | 1 |
| 12 | Sodium Hydroxide | 0.25 | 0.25 | 0.25 | 0.25 |
| 13 | Hydrophilic $TiO_2$ | - | - | - | 0.8 |
| 14 | Hydrophilic Interference Mica *8 | 0.5 | 2 | 2.5 | 2 |
| 15 | Benzyl Alcohol | 0.2 | 0.2 | 0.2 | 0.2 |
| 16 | Perfume | 0.15 | 0.15 | 0.15 | 0.15 |
| 17 | Niacinamide | 3 | 3 | 3 | 3 |
| 18 | Sodium Ascorbyl Phosphate | 1 | 1 | 1 | 1 |
| 19 | Glucosyl Hesperidin | 0.1 | 0.1 | 0.1 | 0.1 |
| Oil Droplets | | | | | |
| 20 | Stearyl dimethicone *10 | - | 0.06 | - | 0.06 |
| 21 | Dimethicone (and) Dimethiconol *11 | - | 0.19 | - | 0.19 |
| 22 | Isopropyl Isostearate | - | - | 0.25 | - |
| 23 | hydrophobic $TiO_2$ *13 | 0.2 | 1 | 1 | 1 |
| *1 Carbopol 980 from Noveon<br>*8 KTZ Xian Vista - 11S2 from Kobo Products Inc<br>*10 DC2503 from Dow Corning Corporation<br>*11 DC1503 from Dow Corning Corporation<br>*13 SA Titanium Dioxide CR-50 from MIYOSHI KASEI CO LTD | | | | | |

The preparation of the cosmetic composition of Comparative Example 1 is as below.

1) Prepare a mixture of water soluble components 1, 3-4, 9-10, 12-13 and 15;
2) Prepare a mixture of oil soluble components comprising 5-8, 11 and 23;
3) Mix those mixtures from step 1) and 2) at a temperature of around 75°C to effect emulsification, and when a homogenous mixture is achieved, cool it down to around 50°C, and add a mixture of less heat resistant components including components 2, 14, 16-19. The composition then can be stored at room temperature.

In the composition so achieved in this Comparative Example 1, the oil ingredients are used to prepare the oil-in-water emulsion, i.e., the cosmetically acceptable carrier. There are no first oil droplets comprising a first oil and a non-interference pigment in Comparative Example 1.

The preparation of the cosmetic composition in Comparative Examples 2-3 is as below.

1) Prepare a mixture of water soluble components 1, 3-4, 9-10, 12-13 and 15;
2) Prepare a mixture of oil soluble components comprising 5-8 and 11;
3) Mix those mixtures from step 1) and 2) at a temperature of around 75°C to effect emulsification, and when a homogenous mixture is achieved, cool it down to around 50°C, and add a mixture of less heat resistant components including water soluble components 2, 16 and 20-22, and components 17;
4) Prepare a blend of separate oils including components 20-21 with hydrophobic non-interference pigment including components 23. Heating can be used to make homogenous mixture of oil and pigment, if needed;
5) Pour the blend of step 4) into the oil-in-water emulsion of step 3) at 30°C, and stirred for 10 minutes at a speed of 1000-1200 rpm/min using a blender coded RW11B from IKA to obtain the oil droplets of the present composition. The composition then can be stored at room temperature.

In the composition so achieved in the Comparative Examples 2-3, there are no first oil droplets formed either, as the condition of blending the first oil and non-interference pigment is strong enough to break the droplet into a very fine particle size, thus no first oil droplet like that of the present invention, which enables the contrast ratio of the cosmetic composition to be less than 10, exists.

Comparative Example 4 composition is prepared according to the same method as that used for preparing the composition of Examples 1-8, except that 0.8% hydrophilic $TiO_2$ is dispersed in step 1) for preparing the oil-in-water emulsion. The incorporation of 0.8% dispersed hydrophilic $TiO_2$ in the hydrophilic phase of the emulsion increase the contrast ratio of the present cosmetic composition to be greater than 10.

COLOR TRANSFORMATION EVALUATION AND CONTRAST RATIO MEASUREMENT

Four products including present compositions of Examples 1 and 2 and comparative compositions of Comparative Examples 2 and 4 are tested among ten panelists for color transformation evaluation. Separately, these four products are measured for their respective contrast ratio following the method described previously in the CONTRAST RATIO MEASUREMENT section.

Measurement results for the color transformation and contrast ratio of the product are summarized in table 3. It can be seen clearly that the two products of present Examples 1 and 2 each has a contrast ratio of less than ten, while for those compositions of the comparative examples 2 and 4, both have a contrast ratio of greater than ten. In terms of color transformation evaluation, all the panelists perceive color transformation of the composition of Examples 1 and 2, but for composition of comparative example 2, nine out ten panelists do not perceive a color transformation and for composition of comparative example 4, none of the panelists perceive a color transformation.

Table 3

| | Ex.1 | Ex.2 | Com Ex.2 | Com Ex. 4 |
|---|---|---|---|---|
| Perceivable pinkish color transformation | 10 | 10 | 1 | 0 |
| No Perceivable pinkish color transformation | 0 | 0 | 9 | 10 |
| Contrast ratio value | 6.6 | 7.4 | 13.3 | 20.3 |

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

Every document cited herein, including any cross referenced or related patent or application, is hereby incorporated herein by reference in its entirety unless expressly excluded or otherwise limited. The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document incorporated by reference, the meaning or definition assigned to that term in this document shall govern.

## Claims

1. A cosmetic composition comprising:

    a) from 0.1% to 5% by weight of a colored interference pigment;
    b) from 0.1% to 10% by weight of a non-interference pigment concentrate, wherein said non-interference pigment concentrate is in a form of "first oil" droplets comprising from 0.01% to 7% by weight of said "first oil" and from 0.09% to 3% by weight of said non-interference pigment;
    c) from 10% to 90% by weight of a cosmetically acceptable vehicle, wherein said cosmetically acceptable vehicle is a "second oil"-in-water emulsion;
    wherein said cosmetic composition has a contrast ratio of 10 or less measured according to the corresponding paragraph in the description, and has a viscosity of at least 40,000 mPa.s measured with a Brookfiled DV-II+ PRO Digital Viscometer with the shear speed of 5 rpm at 25°C, wherein both the "first oil" and "second oil" are selected from a group of oils consisting of hydrocarbon oil, silicone oil, silicone wax, and combinations thereof.

2. The cosmetic composition of claim 1, wherein said colored interference pigment has an absorbance minimum of from 380 to 750 nm.

3. The cosmetic composition of claim 1, wherein said colored interference pigment has a D0.99 volume particle size of from 0.1 μm to 50 μm.

4. The cosmetic composition of claim 1, wherein said non-interference pigment concentrate comprises a non-interference pigment, which is a whitening pigment selected from a group consisting of $TiO_2$, ZnO, ZrO, mica and combinations thereof.

5. The cosmetic composition of claim 1, wherein the contrast ratio is 8 or less.

6. The cosmetic composition of claim 1, wherein said emulsion further comprises from 0.01% to 10% of a thickener selected from the group consisting of acrylamide polymer, carbomer, fatty alcohol, and combinations thereof.

7. The cosmetic composition of claim 1, wherein said emulsion further comprises an emulsifier selected from the group of sorbitan stearate, sucrose cocoate, cetearyl glucoside and cetearyl alcohol, stearic acid, PEG-100 stearate, and combinations thereof.

8. The cosmetic composition of claim 1, comprising 1%-2% by weight of $TiO_2$ coated mica, 1%-5% by weight of the "first oil" droplets comprising 0.5%-2% by weight titanium dioxide and 0.5%-3% by weight of the "first oil" selected from a group consisting of isopropyl isostearate; tocopheyl acetate, a mixture of dimethicone and dimethiconol and combinations thereof.

9. The cosmetic composition of claim 1, wherein the "first oil" has a melting point of no less than 27°C, and the "second oil" has a melting point of from 32°C to 40°C.

10. The cosmetic composition of claim 1, wherein said non-interference pigment concentrate is in a form of beads comprising agar, cellulose and combinations thereof.

11. The cosmetic composition of claim 1, wherein said cosmetic composition is in a form selected from a group consisting of a skin care composition, a hair conditioner, a shaving cream, a personal cleansing product, a color cosmetic composition, and combinations thereof.

12. A method of presenting a color transformation of a cosmetic composition during its application comprising applying the cosmetic composition of any one of claims 1-11.

## Patentansprüche

1. Kosmetikzusammensetzung, umfassend:

    a) von 0,1 Gew.-% bis 5 Gew.-% eines farbigen Interferenzpigments;

b) von 0,1 Gew.-% bis 10 Gew.-% eines Nicht-Interferenzpigmentkonzentrats, wobei das Nicht-Interferenzpigmentkonzentrat in einer Form von "Erstöl"-Tröpfchen vorliegt, umfassend von 0,01 Gew.-% bis 7 Gew.-% des "Erstöls" und von 0,09 Gew.-% bis 3 Gew.-% des Nicht-Interferenzpigments;
c) von 10 Gew.-% bis 90 Gew.-% einer kosmetisch verträglichen Trägersubstanz, wobei es sich bei der kosmetisch verträglichen Trägersubstanz um eine "Zweitöl"-in-Wasser-Emulsion handelt;

wobei die Kosmetikzusammensetzung gemäß dem entsprechenden Absatz in der Beschreibung gemessen ein Kontrastverhältnis von 10 oder weniger aufweist
und eine Viskosität von wenigstens 40.000 mPa, gemessen mit einem Brookfiled DV-II+ PRO Digital-Viskosimeter mit einer Schergeschwindigkeit von 5 U/min bei 25 °C aufweist, wobei sowohl das "Erstöl" als auch das "Zweitöl" ausgewählt sind aus einer Gruppe von Ölen bestehend aus Kohlenwasserstofföl, Silikonöl, Silikonwachs und Kombinationen davon.

**2.** Kosmetikzusammensetzung nach Anspruch 1, wobei das farbige Interferenzpigment ein Absorptionsminimum von 380 bis 750 nm aufweist.

**3.** Kosmetikzusammensetzung nach Anspruch 1, wobei das farbige Interferenzpigment eine D0,99-Volumenteilchengröße von 0,1 μm bis 50 μm aufweist.

**4.** Kosmetikzusammensetzung nach Anspruch 1, wobei das Nicht-Interferenzpigmentkonzentrat ein Nicht-Interferenzpigment umfasst, bei dem es sich um ein Weißpigment handelt, ausgewählt aus einer Gruppe bestehend aus $TiO_2$, ZnO, ZrO, Glimmer und Kombinationen davon.

**5.** Kosmetikzusammensetzung nach Anspruch 1, wobei das Kontrastverhältnis 8 oder weniger beträgt.

**6.** Kosmetikzusammensetzung nach Anspruch 1, wobei die Emulsion ferner von 0,01 % bis 10 % eines Verdickungsmittels umfasst, ausgewählt aus der Gruppe bestehend aus Acrylamidpolymer, Carbomer, Fettalkohol und Kombinationen davon.

**7.** Kosmetikzusammensetzung nach Anspruch 1, wobei die Emulsion ferner einen Emulgator umfasst, ausgewählt aus der Gruppe aus Sorbitanstearat, Saccharosekokoat, Cetearylglucosid und Cetearylalkohol, Stearinsäure, PEG-100-Stearat und Kombinationen davon.

**8.** Kosmetikzusammensetzung nach Anspruch 1, umfassend 1 Gew.-% bis 2 Gew.-% $TiO_2$-beschichteten Glimmers, 1 Gew-% bis 5 Gew-% der "Erstöl"-Tröpfchen, umfassend 0,5 Gew.-% bis 2 Gew.-% Titandioxid und 0,5 Gew.-% bis 3 Gew.-% des "Erstöls" ausgewählt aus einer Gruppe bestehend aus Isopropylisostearat, Tocopherylacetat, einer Mischung aus Dimethicon und Dimethiconol und Kombinationen davon.

**9.** Kosmetikzusammensetzung nach Anspruch 1, wobei das "Erstöl" einen Schmelzpunkt von nicht unter 27 °C aufweist und das "Zweitöl" einen Schmelzpunkt von 32 °C bis 40 °C aufweist.

**10.** Kosmetikzusammensetzung nach Anspruch 1, wobei das Nicht-Interferenzpigmentkonzentrat in Form von Perlen vorliegt, die Agar, Cellulose und Kombinationen davon umfassen.

**11.** Kosmetikzusammensetzung nach Anspruch 1, wobei die Kosmetikzusammensetzung in einer Form vorliegt, die ausgewählt ist aus einer Gruppe bestehend aus einem Hautpflegemittel, einer Haarspülung, einer Rasiercreme, einem Körperreinigungsprodukt, einer farbigen Kosmetikzusammensetzung und Kombinationen davon.

**12.** Verfahren zur Präsentation einer Farbtransformation einer Kosmetikzusammensetzung während ihrer Anwendung, umfassend Anwenden der Kosmetikzusammensetzung nach einem der Ansprüche 1-11.

## Revendications

**1.** Composition cosmétique comprenant :

a) de 0,1 % à 5 % en poids d’un pigment d’interférence coloré ;
b) de 0,1 % à 10 % en poids d’un concentré de pigment sans interférence, dans laquelle ledit concentré de

pigment sans interférence est sous une forme de gouttelettes de « première huile » comprenant de 0,01 % à 7 % en poids de ladite « première huile » et de 0,09 % à 3 % en poids dudit pigment sans interférence ;
c) de 10 % à 90 % en poids d'un véhicule acceptable sur le plan cosmétique, dans laquelle ledit véhicule acceptable sur le plan cosmétique est une émulsion de « deuxième huile »-dans-l'eau ;

dans laquelle ladite composition cosmétique a un rapport de contraste de 10 ou moins mesuré selon le paragraphe correspondant dans la description,
et a une viscosité d'au moins 40 000 mPa.s mesurée avec un viscosimètre digital Brookfiled DV-II+ PRO avec la vitesse de cisaillement de 5 tr/min à 25 °C, dans laquelle tant la « première huile » que la « deuxième huile » sont choisies parmi un groupe d'huiles constitué d'une huile hydrocarbure, une huile de silicone, une cire de silicone, et leurs combinaisons.

**2.** Composition cosmétique selon la revendication 1, dans laquelle ledit pigment d'interférence coloré a un minimum d'absorbance allant de 380 à 750 nm.

**3.** Composition cosmétique selon la revendication 1, dans laquelle ledit pigment d'interférence coloré a une taille de particules en volume D0,99 allant de 0,1 $\mu$m à 50 $\mu$m.

**4.** Composition cosmétique selon la revendication 1, dans laquelle ledit concentré de pigment sans interférence comprend un pigment sans interférence, qui est un pigment de blanchissement choisi dans un groupe constitué de $TiO_2$, ZnO, ZrO, mica et leurs combinaisons.

**5.** Composition cosmétique selon la revendication 1, dans laquelle le rapport de contraste est de 8 ou moins.

**6.** Composition cosmétique selon la revendication 1, dans laquelle ladite émulsion comprend en outre de 0,01 % à 10 % d'un épaississant choisi dans le groupe constitué de polymère acrylamide, carbomère, alcool gras, et leurs combinaisons.

**7.** Composition cosmétique selon la revendication 1, dans laquelle ladite émulsion comprend en outre un émulsifiant choisi parmi le groupe de stéarate de sorbitan, cocoate de saccharose, cétéaryl-glucoside et alcool cétéarylique, acide stéarique, stéarate de PEG-100, et leurs combinaisons.

**8.** Composition cosmétique selon la revendication 1, comprenant 1 % à 2 % en poids de mica enrobé de $TiO_2$, 1 % à 5 % en poids des gouttelettes de « première huile » comprenant 0,5 % à 2 % en poids de dioxyde de titane et 0,5 % à 3 % en poids de la « première huile » choisie dans un groupe constitué de l'isostéarate d'isopropyle ; l'acétate de tocophérol, un mélange de diméthicone et de diméthiconol et leurs combinaisons.

**9.** Composition cosmétique selon la revendication 1, dans laquelle la « première huile » a un point de fusion d'au moins 27 °C, et la « deuxième huile » a un point de fusion allant de 32 °C à 40 °C.

**10.** Composition cosmétique selon la revendication 1, dans laquelle ledit concentré de pigment sans interférence est sous une forme de perles comprenant de l'agar-agar, de la cellulose et leurs combinaisons.

**11.** Composition cosmétique selon la revendication 1, dans laquelle ladite composition cosmétique est sous une forme choisie dans un groupe constitué d'une composition de soin de la peau, d'un conditionneur pour les cheveux, d'une crème de rasage, d'un produit de nettoyage corporel, d'une composition cosmétique de couleur, et de leurs combinaisons.

**12.** Procédé de présentation d'une transformation de couleur d'une composition cosmétique pendant son application comprenant l'application de la composition cosmétique selon l'une quelconque des revendications 1 à 11.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2009112970 A
- EP 2169040 A1


### Non-patent literature cited in the description


- Standard Practice for Particle Size Analysis of Particulate Substances in the Range of 0.2 to 75 Micrometers by Optical Microscopy. *ASTM,* 1993, vol. 14.02
- North American Edition. McCutcheon's, Detergents and Emulsifiers. Allured Publishing Corporation, 1986
- the chromameter manual. 1988